# EUROPÄISCHE PATENTSCHRIFT

(11) **EP 2 191 265 B1**
(45) Veröffentlichungstag und Bekanntmachung des Hinweises auf die Patenterteilung: **28.11.2012**
(21) Anmeldenummer: 08803365.9
(22) Anmeldetag: 29.08.2008
(51) Int. Cl.: G01N 33/28, G01N 21/35

(54) **KRAFTSTOFFSYSTEM FÜR EINE SCHWIMMENDE EINRICHTUNG UND VERFAHREN ZU DESSEN BETREIBEN**
FUEL SYSTEM FOR A FLOATING UNIT, AND METHOD FOR THE OPERATION THEREOF
CIRCUIT DE CARBURANT CONCU POUR UNE UNITE FLOTTANTE ET PROCEDE DE FONCTIONNEMENT ASSOCIE

(30) Priorität: 19.09.2007 DE 102007044970; 02.05.2008 DE 102008021899
(43) Veröffentlichungstag der Anmeldung: 02.06.2010
(73) Patentinhaber: Siemens Aktiengesellschaft, 80333 München (DE)
(72) Erfinder: MÜLLER-SCHWENN, Hans Bernhard, 22529 Hamburg (DE); FLEISCHER, Maximilian, 85635 Höhenkirchen (DE); LAMPE, Uwe, 21614 Buxtehude (DE); WIESNER, Kerstin, 85640 Putzbrunn (DE)
(86) Internationale Anmeldenummer: PCT/EP2008/061367
(87) Internationale Veröffentlichungsnummer: WO 2009/037089

(56) Entgegenhaltungen:
- WO-A-96/00380
- WO-A-2007/042051
- WO-A-2007/093500

## Beschreibung

Die Erfindung betrifft ein Kraftstoffsystem für eine schwimmende Einrichtung und ein Verfahren zum Betreiben desselben.

Schwimmende Einrichtungen im Sinne des vorliegenden Schriftstückes sind z.B. Seefahrzeuge wie Schiffe oder Tauchboote oder auch kraftstoffverbrauchende Offshoreanlagen wie Bohrinseln oder Windkraftanlagen. Der Einfachheit halber wird im Folgenden stellvertretend hierfür von Schiffen gesprochen.

Kraftstoffe für Schiffe (in diesem Zusammenhang häufig auch als "Treibstoffe" oder "Brennstoffe" bezeichnet) zeichnen sich durch einen hohen Schwefelgehalt von bis zu 5 Gew.% aus. Da der Schiffsverkehr aufgrund des zunehmenden Welthandels in den letzten Jahren sehr stark zugenommen hat, ist sein Anteil an der Umweltverschmutzung in Häfen und küstennahen Gewässern deutlich angestiegen. Dabei sind insbesondere die schwefelhaltigen Abgasbestandteile wie SO₂ besonders schädigend. Daher gibt es seit einiger Zeit Bestrebungen, über eine Begrenzung des Schwefelgehaltes der Kraftstoffe die Schwefelemissionen durch Schiffe zu begrenzen. Für die SECAS (SOₓ-EMISSIONS CONTROLLED AREAS) Nordsee und die Ostsee ist z.B. vorgeschrieben, dass nur noch Kraftstoffe mit einem maximalen Schwefelgehalt von 1,5 Gew.%, und in Häfen sogar nur noch schwefelfreie Kraftstoffe (Gehalt < 0,1 Gew.%) verwendet werden dürfen. An der Westküste der USA gelten ähnliche Regelungen. Bei der Fahrt in diesen Gewässern werden Schiffe kontrolliert, ob nur Kraftstoffe mit einem Gehalt unterhalb des vorgeschriebenen Limits aktuell eingesetzt werden. Bei Zuwiderhandlung drohen empfindliche Strafen.

Auf den Weltmeeren außerhalb der gesetzlich festgelegten Seegebiete darf weiterhin Kraftstoff mit beliebigem Schwefelgehalt verbrannt werden. Da schwefelarmer Kraftstoff teurer ist als Kraftstoff mit hohem Schwefelgehalt, werden Schiffe auf dem größten Teil der Fahrt mit billigem schwefelhaltigen Kraftstoff betrieben und nur in den bestimmten Gebieten schwefelarme Kraftstoffe eingesetzt. Das bedeutet, dass Schiffe künftig Kraftstoffe mit unterschiedlichen Qualitäten bunkern.

Die EU - Richtlinie 2005/33/EG sieht vor,
- ab 11. August 2006 Einführung eines Schwefelgrenzwertes von 1,5 % für Schiffskraftstoffe, die von Schiffen in der Ostsee und ab 11. August 2007 in der Nordsee und im Ärmelkanal verwendet werden, um die Versauerung zu reduzieren und die Luftqualität zu verbessern,
- ab 11. August 2006 Einführung eines Schwefelgrenzwertes von 1,5 % für Schiffskraftstoffe, die von Fahrgastschiffen im Linienverkehr von oder nach einem Gemeinschaftshafen verwendet werden, damit die Luftqualität verbessert und zur Sicherstellung einer EU-weiten Versorgung mit schwefelarmen Schiffskraftstoffen eine ausreichende Nachfrage geschaffen wird,
- Einführung eines Schwefelgrenzwertes von 0,1 % ab 1. Januar 2010 für Schiffskraftstoffe, die von Schiffen auf den Binnenwasserstraßen und an Liegeplätzen für Dieselmotoren verwendet werden, damit die Luftqualität an Häfen und Binnenwasserstraßen verbessert wird,
- in Abweichung der oben beschriebenen Grenzwerte für Schweröle Erteilung einer Erlaubnis an Schiffe für den Einsatz einer genehmigten emissionsmindernden Technologie, sofern diese Schiffe ständig zumindest gleichwertige Emissionsminderungen erzielen und ausführlich dokumentiert ist, dass alle Abfallströme, die in geschlossene Häfen und Flussmündungen abgelassen werden, keine Auswirkungen auf die Ökosysteme haben,
- das ordnungsgemäße Führen von Logbüchern mit Angaben zur Kraftstoffumstellung wird zur Auflage dafür, dass Schiffe Häfen der EU-Gemeinschaft anlaufen können,
- der Schwefelgehalt aller im Hoheitsgebiet der Mitgliedstaaten der EU verkauften Kraftstoffe muss vom Lieferanten auf einem von einer Probe begleiteten Lieferschein vermerkt werden,
- das von Schiffskraftstoffen Proben zu entnehmen und auf ihren Schwefelgehalt zu prüfen sind.

Ein schwerwiegendes Problem ist, dass die Kraftstoffqualität weltweit sehr unterschiedlich und nicht durch allgemeingültige Gesetze oder Normen festgelegt ist. Insbesondere ist z.B. in Häfen in Asien, Südamerika oder Afrika damit zu rechnen, dass der Kraftstoff mit falschen Zertifikaten verkauft wird, d.h. der Schwefelgehalt ist in der Regel höher, als im Zertifikat bescheinigt wird. Da bei Kontrollen in den emissionslimitierten Gebieten nicht nur die Zertifikate eingesehen werden, sondern auch direkte Proben des Kraftstoffes genommen und untersucht werden, können erhebliche Probleme auf den Schiffsführer zukommen, wenn ein zu hoher Schwefelgehalt festgestellt wird (Geldstrafen, Entsorgung des falschen Kraftstoffes usw.).

Zum Betreiben eines Schiffes war bisher die Kenntnis des Schwefelgehalts des Kraftstoffes nicht notwendig, da eine Begrenzung der Schwefelemissionen für Schiffe in bestimmten Seegebieten erst seit kurzer Zeit umgesetzt wird. Die EU hat erst 2003 Maßnahmen zur Begrenzung des Schwefelgehaltes beschlossen, deren Umsetzung stufenweise bis zum Jahr 2010 vorgesehen ist. Für das Jahr 2007 ist eine Absenkung des Schwefelgehaltes auf 1,5 Gew.% für den Kraftstoff von Seeschiffen (gilt für die SECAS Nord - und Ostsee) vorgesehen. Der Schwefelgehalt wird heute in der Regel nur bei der Produktion des Kraftstoffes in der Raffinerie gemessen. Der Schiffsführer muss dann auf die Richtigkeit des Zertifikats vertrauen, ohne es jedoch selbst überprüfen zu können.

Aus [Innov-X-Systems / Maersk Fluid Technology, "On-Board Elemental XRF Analysis of Oils & Additives", 2006] ist ein "Sea-Mate (TM)" genanntes Gerät bekannt, welches z.B. auf einem Schiff betreibbar ist und Proben von Kraftstoffen hinsichtlich ihres Schwefelgehalts untersuchen kann. Die Probenahme und anschließende Analyse ist jedoch umständlich und zeitraubend.

Aus WO 2007/093500 A1 ist ein Kraftstoffsystem für ein Schiff mit einem Kraftstoffweg und einer darin angeordneten Messeinrichtung zur Bestimmung des Schwefelgehaltes des Kraftstoffes bekannt.

Es ist deshalb Aufgabe vorliegender Erfindung, ein verbessertes Kraftstoffsystem für eine schwimmende Einrichtung und ein verbessertes Verfahren zu deren Betreiben anzugeben.

Hinsichtlich des Kraftstoffsystems wird die Aufgabe der Erfindung gelöst durch ein solches für einen Betrieb einer schwimmenden Einrichtung, sprich Schiff, mit folgenden Merkmalen: Das Kraftstoffsystem umfasst einen Kraftstoffweg, der sich von einem Tankstutzen zum Betanken des Schiffes bis zu einem auf dem Schiff befindlichen Motor erstreckt. Erfindungsgemäß ist im Kraftstoffweg eine Messeinrichtung angeordnet. Diese wird von zumindest einem Teil des Kraftstoffes durchflossen, der sich auf dem Kraftstoffweg bewegt. Mit anderen Worten ist die Messeinrichtung z.B. seriell in den Kraftstoffweg geschaltet oder nach Art eines Bypasses an diesen parallel angeschlossen. Die Messeinrichtung ist damit vom entlang des Kraftstoffweges strömenden Kraftstoffes durchströmbar bzw. durchströmt. Die Messeinrichtung ist eine solche zur Messung eines Schwefelgehaltes des die Messeinrichtung durchströmenden Kraftstoffes.

Dabei weist das Kraftstoffsystem erfindungsgemäß mindestens zwei Kraftstofftanks für Kraftstoffe unterschiedlichen Schwefelgehaltes, eine Mischeinrichtung zur Mischung von Kraftstoffen der Kraftstofftanks und eine mit der Messeinrichtung zusammenwirkende Regeleinrichtung zur Regelung des Mischungsverhältnisses der Kraftstoffe auf.

Durch die Erfindung lässt sich also eine ständige Messung, mit anderen Worten eine Online-Messung, der Kraftstoffqualität bzgl. Schwefelgehalt im Kraftstoffweg des Schiffes realisieren. Der Schiffsführer ist damit über die Kraftstoffqualität in demjenigen Abschnitt des Kraftstoffweges informiert, wo die Messung stattfindet. Für den Ort der Messung existieren viele - weiter unten erläuterte und auch miteinander kombinierbare - Möglichkeiten. Es muss keine aufwändige Probenahme an einer bestimmten Stelle des Kraftstoffweges und Analyse an einem anderen Ort, z.B. in einem Labor erfolgen. Der Schwefelgehalt ist direkt vor Ort und ständig ermittelbar.

Da mit einem Online-Messsystem der Schwefelgehalt des Kraftstoffes jederzeit einfach ermittelbar ist, können die Forderungen an die Qualität und die Kontrolle der Qualität des Schiffskraftstoffes gemäß der EU - Richtlinie 2005/33/EG unaufwändig erfüllt werden.

Durch die Ausstattung des Kraftstoffsystems mit mindestens zwei Kraftstofftanks für Kraftstoffe unterschiedlichen Schwefelgehaltes ist eine Umschaltung zwischen beiden Kraftstoffen z.B. zwischen offener See und küstennahen Gewässern möglich.

Durch die Mischeinrichtung zur Mischung von Kraftstoffen der Kraftstofftanks und die mit der Messeinrichtung zusammenwirkende Regeleinrichtung zur Regelung des Mischungsverhältnisses der Kraftstoffe kann z.B. ein Kraftstoffblending erreicht werden, so dass der Schwefelgehalt im zu verbrennenden Kraftstoff immer gerade unterhalb der erlaubten Grenzwerte liegt.

Der Mischer kann hierbei auch ein einfacher Umschalter für die beiden verschiedenen Kraftstoffe sein, wenn ein variables Blending nicht gewünscht ist.

In einer vorteilhaften Ausführungsform der Erfindung umfasst die Messeinrichtung eine Lichtquelle zur Bestrahlung des Kraftstoffes mit Licht. Außerdem umfasst sie einen Detektor für Licht, welches - ausgehend von der Lichtquelle - den Kraftstoff durchstrahlt hat oder vom Kraftstoff reflektiert wurde. Mit anderen Worten wird der Schwefelgehalt durch eine Transmissions- oder Reflexionsmessung am Kraftstoff bestimmt. Eine solche Messanordnung ist leicht in den Kraftstoffweg einbaubar, z.B. in eine Kraftstoffleitung.

Für die genannte Messung eignet sich z.B. die IR-Spektroskopie. In einer vorteilhaften Ausgestaltung der Erfindung kann daher die Lichtquelle eine IR-Lichtquelle sein. Insbesondere kann dann die Lichtquelle im NIR- oder MIR-Bereich strahlen.

In einer weiteren bevorzugten Ausführungsform ist dann der Detektor ein IR-Spektrometer. Dieses eignet sich besonders im Zusammenwirken mit der o.g. Lichtquelle.

Grundlage der IR - Spektroskopie ist die Absorption des IR - Lichtes, wodurch Molekülschwingungen und/oder -rotationen angeregt werden. Man unterscheidet die Bereiche des Fernen Infrarot (FIR, Wellenlänge: 30 - 3000 µm), des Mittleren Infrarot (MIR, Wellenlänge: 2,5 - 30 µm) und des Nahen Infrarot (NIR, Wellenlänge: 0,8 - 2,5 µm). Im Bereich des FIR werden die Molekülrotationen angeregt, im Bereich des MIR die molekularen Grundschwingungen und im Bereich des NIR die Ober- und Kombinationsschwingungen. Eingesetzt werden für die Zwecke der Analytik bevorzugt die MIR - und die NIR - Spektroskopie. Die Basis dafür ist, dass grundsätzlich die Schwingungsfrequenz und damit die Wellenlänge des absorbierten IR - Lichtes abhängt von der spezifischen Stärke der chemischen Bindung und der Masse der schwingenden Atome bzw. Atomgruppen.

Die Intensität hängt ab von der Stärke des Dipolmoments der anzuregenden Atomgruppe und der Konzentration. Die IR - Spektroskopie gibt daher Auskunft über die qualitative Natur der absorbierenden Spezies und deren quantitativen Anteil in einer Mischung.

Die MIR - Spektroskopie hat den Vorteil, dass sie Auskunft über einzelne lokalisierte Atomgruppen gibt, die einer bestimmten chemischen Spezies zugeordnet werden kann. Dies erleichtert deren Identifikation. Insbesondere organische Schwefelverbindungen lassen sich leicht identifizieren, vor allem aufgrund der im Vergleich zu anderen Atomen einer organischen Verbindung hohen Masse des Schwefelatoms (Verschiebung der Absorption zu größeren Wellenlängen). Nachteilig bei der MIR - Spektroskopie ist, dass dies Verfahren sich nur mit großem Aufwand als on - line Messverfahren realisieren lässt. Dies betrifft insbesondere die benötigten Messzellen und Lichtleitfasern.

Die NIR - Spektroskopie erlaubt nur ausnahmsweise eine Zuordnung der gemessenen Absorptionen zu bestimmten Molekülen oder Molekülgruppen, weiterhin ist die Absorption der NIR - Strahlung deutlich geringer als der MIR - Strahlung. Dies ist jedoch vorteilhaft, da die geringere Absorption durch eine grö-βere optische Weglänge ausgeglichen werden kann. Von Vorteil ist, dass die NIR - Spektroskopie mit deutlich gegenüber der MIR - Methode reduziertem Aufwand als on -line Methode realisiert werden kann. Dies betrifft vor allem die Messzellen und Lichtleiter. Auch die benötigten größeren optischen Weglängen sind vorteilhaft, da sie einen Aufbau erlauben, der weniger empfindlich auf Verschmutzung und leichter zu reinigen ist.

Da der Schwefelgehalt in Schiffskraftstoffen von einer Reihe organischer Schwefelverbindungen, zumeist Thiole (CₓH_{y}-SH), Thioether (Cₓ₁H_{y1}-S-Cₓ₂H_{y2}), Heteroaromaten (z.B. C₄H₄S Thiophen) oder Disulfide (Cₓ₁H_{y1}-S-S-Cₓ₂H_{y2}) hervorgerufen wird, werden bevorzugt die Lage und Intensitäten von mehreren Absorptionsbanden in eine quantitative Bestimmung des Gesamtschwefelgehaltes einbezogen werden. Dies ist unter der Verwendung von multivariaten Auswerteverfahren oder einer Kombination von multivariaten Methoden mit Neuronalen Netzen möglich. Eine Kalibration der IR - Spektren kann unter zu Hilfenahme von im Labor mit Standardmethoden bestimmten Schwefelkonzentrationen im Schiffskraftstoff erfolgen.

In einer weiteren vorteilhaften Ausführungsform kann die Messeinrichtung eine Auswerteeinheit zur multivariaten Auswertung des den Kraftstoff durchstrahlenden oder vom Kraftstoff reflektierten Lichtes umfassen.

Die IR - Spektroskopie, insbesondere in Verbindung mit multivariater Auswertung (Chemometrie, ANN) der Spektren, erlaubt eine Online-Messung des Schwefelgehaltes von Schiffskraftstoff beim Bunkern des Kraftstoffs ebenso wie beim Betrieb des Schiffes.

In einer weiteren vorteilhaften Ausführungsform kann die Messeinrichtung in einem zumindest beim Betrieb eines Motors der schwimmenden Einrichtung von Treibstoff durchströmten Abschnittes des Kraftstoffweges angeordnet sein. Mit einer Online-Messung während des Betriebes kann die Schiffsführung gleichzeitig nachweisen, dass vor Einfahrt in das emissionsbegrenzte Gebiet rechtzeitig die Versorgung der Maschine auf den schwefelarmen Kraftstoff umgestellt wurde.

In einer weiteren vorteilhaften Ausführungsform der Erfindung kann im Kraftstoffsystem eine Protokolliervorrichtung zur Protokollierung von durch die Messeinrichtung ermitteltem Schwefelgehalt vorhanden sein. Ein derartiges Protokoll kann vom Schiffsführer z.B. gegenüber Behörden als Beweismittel benutzt werden, um die korrekte Kraftstoffqualität nachzuweisen.

Die Online-Messung der Kraftstoffqualität lässt also eine Betriebsweise zu, bei der schwefelarmer und schwefelhaltiger Kraftstoff kostengünstig gemischt werden, so dass schließlich ein Kraftstoff mit der maximal erlaubten Schwefelkonzentration verbrannt wird. Die online gemessenen Daten können also unmittelbar zur Prozessregelung eingesetzt werden sowie für die erforderlichen z.B. behördlichen Nachweise protokolliert werden.

Gemäß der Erfindung kann eine Messsonde zur Bestimmung des Schwefelgehaltes also z.B. in die Bunkerleitungen von Schwer- und Dieselöl etwa 1m nach dem Bunkerflansch, in die Brennstoffleitung vor dem Hauptmotor, in die Brennstoffleitungen vor Dieselgeneratorsätzen und eventuell vor und nach Misch- und Blendungseinrichtungen bzw. -aggregaten in Brennstoffsysteme für Schweröl eingebaut werden.

Die Erfindung ermöglicht also eine direkte Kontrolle des Schwefelgehaltes des Kraftstoffs beim Bunkern. Dies ist insbesondere in Häfen außerhalb der EU und der USA von Bedeutung. Der Schiffsführer kann sofort die Zufuhr von Kraftstoff mit zu hohem Schwefelgehalt abbrechen. Der Schiffsführer kann gegenüber den Behörden anhand des automatisch erstellten Bunkerprotokolls nachweisen, dass Kraftstoff mit einem Schwefelgehalt unterhalb des gesetzlichen Limits an Bord ist. Bei Mischbetrieb (schwefelhaltiger Kraftstoff auf See, schwefelarmer Kraftstoff in Küstengewässern) kann die erfolgte Umschaltung der Kraftstoffsorten bei der Einfahrt in die Gebiete mit Emissionsbegrenzung überprüft und durch das Protokoll nachgewiesen werden. Damit ist es möglich, große Strecken mit preiswerten schwefelhaltigen Kraftstoffen zu fahren und kann gleichzeitig der Gebrauch des vorgeschriebenen Kraftstoffs für die Küstengewässer mit Emissionsbegrenzung nachgewiesen werden. Auf der Basis der Online-Messung kann ein Kraftstoffblending (Mischung von schwefelhaltigem / schwefelarmem Kraftstoff) für den Betrieb des Schiffes durchgeführt werden. Dies erlaubt, dass im Wesentlichen nur zwei Sorten von Kraftstoff benötigt werden (schwefelhaltiger und schwefelfreier).

Alle erlaubten Zwischenstufen von Schwefelkonzentrationen können dann kostengünstig durch ein entsprechendes Mischen der Kraftstoffe eingestellt werden.

Der Einsatz der IR - Spektroskopie bei Verwendung als Online-Methode zur Bestimmung der Schwefelkonzentration im Kraftstoff, ergibt den Vorteil, dass die Verwendung des richtigen Kraftstoffs nachgewiesen werden kann. Damit entfallen teure Kontrollanalysen im Labor. Der Schiffseigner kann sich vor den negativen Folgen einer Bunkerung mit falschem Kraftstoff schützen (u.a. Strafzahlungen, Entsorgung des falschen Kraftstoffes). Das Schiff kann immer die preiswertesten Kraftstoffe einsetzen. Die Umweltbelastung durch Schwefelemissionen in den küstennahen Gewässern und Häfen kann gesenkt werden.

Hinsichtlich des Verfahrens wird die Aufgabe gelöst durch ein Verfahren zum Betreiben eines Kraftstoffsystems einer schwimmenden Einrichtung, das einen sich von einem Tankstutzen bis zu einem Motor erstreckenden Kraftstoffweg aufweist. Beim Verfahren wird mit einer im Kraftstoffweg angeordneten Messeinrichtung der Schwefelgehalt in demjenigen Teil des Kraftstoffes gemessen, der die Messeinrichtung durchströmt. Hierbei wird die Messeinrichtung von zumindest einem Teil des Kraftstoffes durchströmt, der entlang des Kraftstoffweges strömt. Weiterhin werden beim Betrieb der schwimmenden Einrichtung mindestens zwei Kraftstoffe unterschiedlichen Schwefelgehaltes gemischt, und es wird anhand des gemessenen Schwefelgehalts der Schwefelgehalt des gemischten Kraftstoffes auf einem vorgebbaren Wert gehalten.

Das Verfahren zusammen mit seinen Vorteilen wurde bereits im Zusammenhang mit dem Kraftstoffsystem erläutert.

Bevorzugte Verwendungen des erfindunsggemäßen Verfahrens bzw. der erfindungsgemäßen Vorrichtung liegen deshalb bei der Messung von Kraftstoffen beim Betrieb eines Schiffes mit unterschiedlichen Kraftstoffen und beim Kraftstoffblending beim Betrieb eines Schiffes.

Für eine weitere Beschreibung der Erfindung wird auf die Ausführungsbeispiele der Zeichnungen verwiesen. Es zeigen, jeweils in einer schematischen Prinzipskizze:
- FIG 1: das MIR Spektrum von Schiffsdiesel mit 0,16% Schwefelgehalt,
- FIG 2: das NIR-Spektrum des Schiffsdiesels aus FIG 1,
- FIG 3: einen Ausschnitt aus dem Kraftstoffweg eines Schiffes mit einer erfindungsgemäßen Messeinrichtung mit Transmissionsdurchflusszelle,
- FIG 4: eine Anordnung gemäß FIG 3 mit einer Reflexionstauchzelle (a) und einer ATR-Messzelle (b),
- FIG 5: ein Ablaufdiagramm für ein für die Berechnung des Schwefelgehaltes im Schiffskraftstoff benötigtes Kalibrationsmodell,
- FIG 6: ein Blendungssystem für Schweröl mit Gasöl.

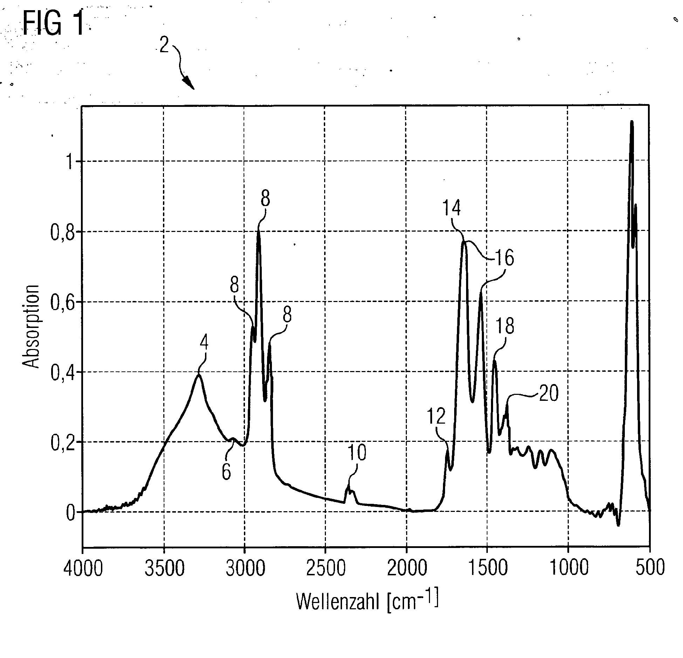

FIG 1 zeigt ein Diagramm 2, welches das MIR - Spektrum von Schiffsdiesel mit 0.16% Schwefel wiedergibt. Das Spektrum wurde mit einem FTIR - Gerät (Bruker ifs 66v) im Bereich einer Wellenzahl zwischen 4000 - 500 cm⁻¹ bei einer Auflösung von 4 cm⁻¹ mit der ATR - Technik aufgenommen, aufgetragen auf der Abszisse des Diagramms 2. Die Ordinate zeigt die Absorption in willkürlichen Einheiten. Zu sehen sind im Einzelnen die OH- und NH-Valenz 4, die C=C-H₂-Valenz 6, die CH-Valenz 8, die Stelle von CO₂ 10, d.h. Luft, die C=O-Valenz 12 bzw. Keton Aldehyd, die C=C Stelle 14, Aromat 16, die CH-Deformation 18 und die -CH₃-symmetrische Deformation 20.

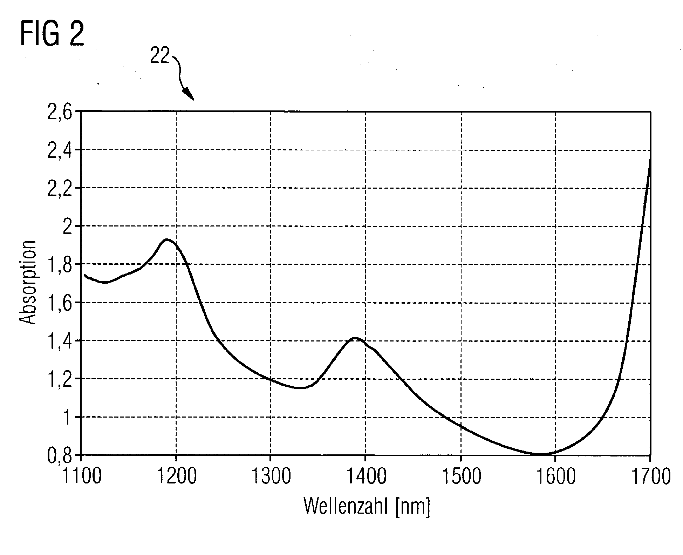

FIG 2 zeigt in einem Diagramm 22 das NIR - Spektrum der gleichen Probe wie in FIG 1. Das Spektrum wurde mit einem NIR - Gerät der Firma Boehringer Ingelheim MicroParts im Bereich einer Wellenlänge (Abszisse) zwischen 1100 - 1700 nm als Transmissionsmessung aufgenommen. Auch hier zeigt die Ordinate die Absorption.

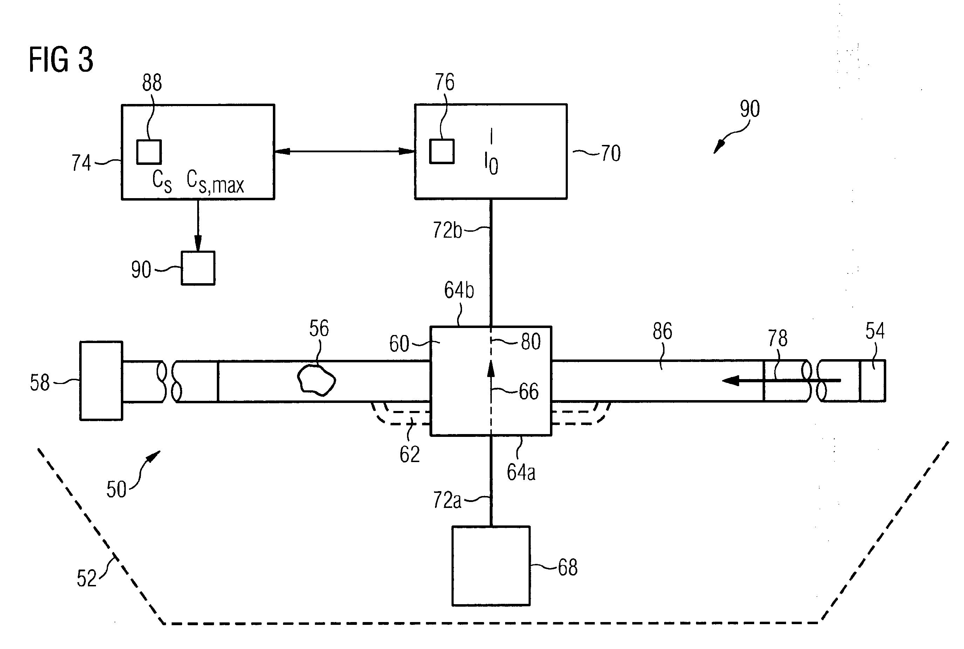

FIG 3 zeigt stark vereinfacht einen Kraftstoffweg 50 in einem Schiff 52, welcher sich von einem Tankstutzen 54 des Schiffes 52 bis zu dessen mit Diesel 56 - stellvertretend für beliebige Kraftstoffe wie Diesel, Schweröl, Gasöl etc. - betriebenen Motor 58 fortsetzt. Im Schiff 52 ist ein Messsystem 90 installiert. Dieses ist wie folgt ausgestaltet: Im Kraftstoffweg ist eine als Transmissionsdurchflusszelle ausgebildete Messzelle 60 angeordnet. Im Beispiel ist der Abschnitt des beim Bunkervorgang durchströmten Zuflussrohres 86 gezeigt. Diese befindet sich im Hauptstrom des Kraftstoffs 56 oder alternativ - gestrichelt dargestellt - in einer By-Pass-Leitung 62 und wird vom Diesel 56 (bzw. alternativ einem Teil dessen) durchflossen. Der Pfeil 78 zeigt die Flussrichtung des Diesels 56.

Die Messzelle 60 weist zwei gegenüberliegende Fenster 64a,b auf. Diese bestehen bevorzugt aus Quarzglas oder Saphir, die optische Weglänge wird bevorzugt zwischen 0,1 und 10 mm eingestellt, je nach der verwendeten Wellenlänge des Lichtes 66. Dieses wird von einer auf der einen Seite der Messzelle 60 angeordneten Lichtquelle 68 erzeugt. Die Lichtquelle 68 strahlt im NIR - Bereich. Hierfür wird vorzugsweise eine Wolfram - Halogen - Lampe benutzt, alternativ für den Bereich des MIR z. B. ein Globar. Gegenüber der Lichtquelle 68 ist ein IR - Spektrometer 70 angeordnet, vorzugsweise ein Mikrospektrometer für den NIR - Bereich oder den MIR - Bereich.

Das Licht wird bevorzugt durch optische Fasern 72a,b von der Lichtquelle 68 zur Messzelle 60 und von dort zum Spektrometer 70 geleitet. Alternativ können Lichtquelle 68 und Spektrometer 70 auch direkt an die Messzelle angeflanscht werden, so dass die opt. Fasern 72a,b entfallen. Ein Computer 74 dient zu Steuerung des Spektrometers 70 und zur Auswertung der von diesem gemessenen Spektren 76.

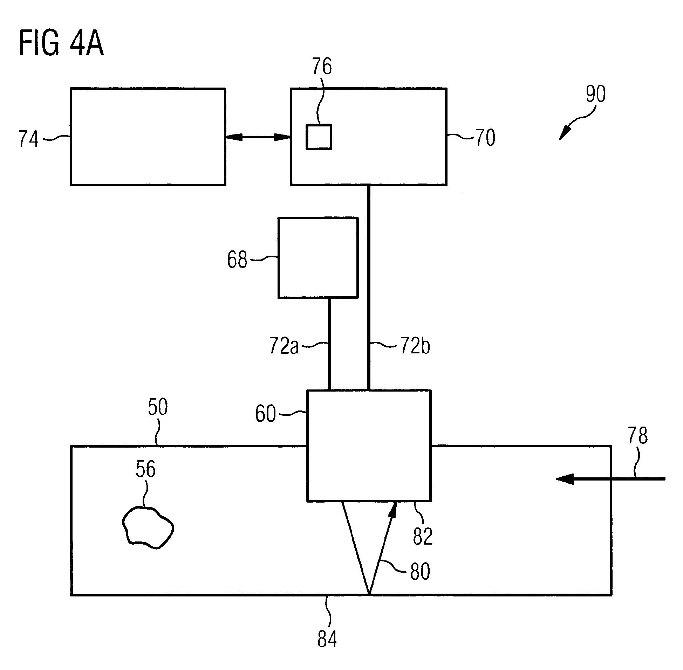

FIG 4a zeigt ein alternatives Messsystem 90 zur FIG 3, bei dem als Messzelle 60 eine Reflexionstauchzelle verwendet wird. Der optische Weg 80 durch den Diesel 56 wird hier durch den Abstand zwischen Austrittsfenster 82 der Messzelle 60 und Spiegel 84 eingestellt.

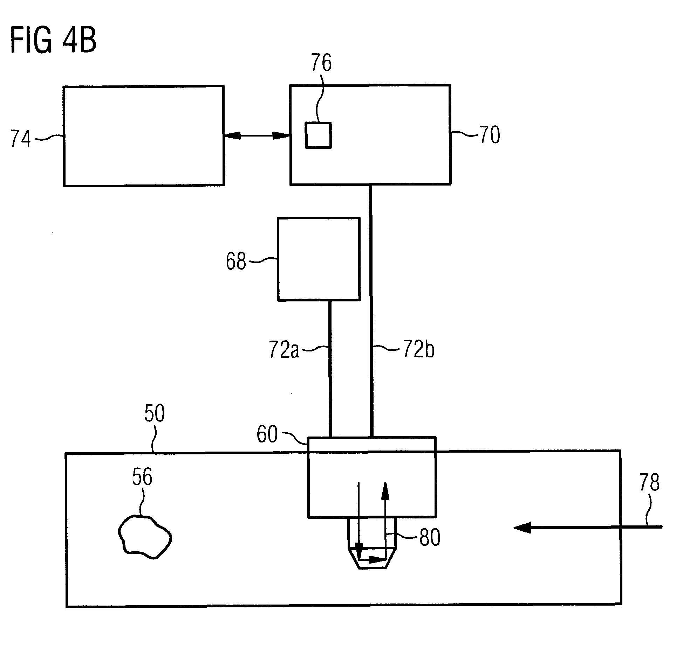

FIG 4b zeigt ein alternatives Messsystem zu den Figs. 3 und 4a, bei dem eine ATR-Zelle als Messzelle 60 verwendet wird. Dabei taucht die ATR-Messzelle in den Kraftstoffweg 50 bzw. den Diesel 56 ein. Der optische Weg 80 wird durch die Zahl der Reflexionen , nämlich mindestens drei, festgelegt.

Als Mikrospektrometer 70 bieten sich zwei unterschiedliche Gerätetypen an:
- Zeilenspektrometer, bei denen mit einem festen mikromechanischen Reflexionsgitter die spektrale Dispersion des Lichtes erreicht wird. Ein Beispiel ist das NIR Gerät von Böhringer Ingelheim MicroParts, bei dem das Reflexionsgitter durch die LIGA - Technik (Lichtinduzierte Galvanische Umformung) hergestellt wird.
- Mikrospektrometer mit bewegten Mikrospiegeln (MOEMS: Mikro - Optisch - Elektrische System), bei denen die Dispersion des Lichtes durch ein Reflexionsgitter aus verspiegeltem Silizium erzeugt wird. Ein Beispiel ist das MOEMS - IR Gerät der Firma ColourControl.

In den FIGs 3 oder 4a,b läuft eine Messung wie folgt ab:
1. Vor Beginn des Bunkervorganges, also der Aufnahme von Diesel 56 durch den Tankstutzen 54 in das Schiff 52, wird bei leerem Zuflussrohr 86 die Intensität I₀ (Lampenspektrum) ermittelt. Es wird geprüft, ob eine erforderliche mindeste Lichtintensität I₀ > I₀, ₘᵢₙ erreicht wird. Wird diese unterschritten, gibt der Steuerrechner 74 eine Fehlermeldung aus und fordert zum Wechsel der Lichtquelle 68 bzw. zum Reinigen des optischen Weges 80 auf.
2. Nach Beginn des Durchflusses des Diesels 56 beginnt die Messung der Intensität I, die mit Hilfe der Größe I₀ in die Absorption umgerechnet wird.
3. Aus der gemessenen Absorption und dem im Computer 74 eingespeichertem Kalibrationsmodell 88 wird der Schwefelgehalt C_{S} des Diesels 56 berechnet. Dieser Wert kann unmittelbar angezeigt werden und/oder in einem Bunkerprotokoll 90 abgespeichert werden. Es kann alternativ vorgesehen werden, dass bei Überschreiten von einem festgelegten Grenzwert C_{S}>C_{S},ₘₐₓ der Bunkervorgang automatisch unterbrochen wird.

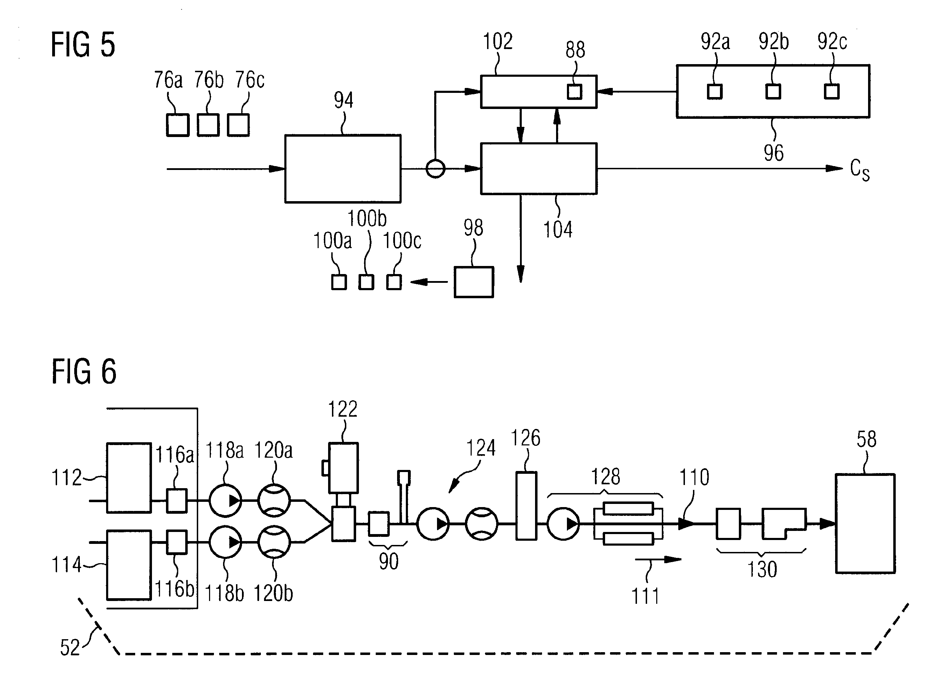

Das für die Berechnung des Schwefelgehaltes C_{S} im Diesel 56 benötigte Kalibrationsmodell 88 kann auf folgendem, in FIG 5 gezeigten Weg erstellt werden:
1. Es werden die Absorptionsspektren 76a-c von verschiedenen Schiffskraftstoffen, also Diesel 56, ermittelt. Der Schwefelgehalt C_{S} dieser Kraftstoffe ist vorab bereits mit bekannten Labormethoden ermittelt worden, also vorab bekannt und in Laborwerten 92a-c gespeichert. Der optische Aufbau (Art der Messsonde, optische Weglänge, Spektrometer- und Lichtquellentyp) und die Umgebungsbedingungen (Temperatur, Druck) müssen vergleichbar denen sein, unter denen das Messsystem 90 auf Schiffen 52 später eingesetzt werden soll. Die als Referenz benutzte Laboranalyse, also die Laborwerte 92a-c müssen einen möglichst geringen Messfehler haben, da dieser Messfehler in das Kalibrationsmodell 88 als Fehler eingeht und nicht unterschritten werden kann.
2. Nun beginnt eine Vorverarbeitung 94. Dort werden Fehlmessungen entfernt.
3. Die Spektren 76a-c werden hierbei geglättet bzw. es werden Ableitungen gebildet.
4. Die Spektren 76a-c werden auch in mehrere spektrale Bereiche eingeteilt.
5. Die Spektren 76a-c werden in Trainings- und Validiersatz nach den üblichen Methoden (z.B. Venetian Blind, Leaveone-out) aufgeteilt.
6. Die Spektren 76a-c werden normiert. Es bieten sich beispielsweise folgende Verfahren an: Skalieren, Zentrieren, Minimum-Maximum, Vektornormierung, orthogonale Signalkorrektur. Die Normierungsfaktoren des Trainingssatzes werden benutzt, die Spektren des Validierungssatzes umzurechnen.
7. In einer Auswertung 104 werden die Spektren 76a-c ausgewertet. Zwischen den normierten Absorptionsspektren 76a-c und den Schwefelkonzentrationen der Laborwerte 92a-c aus der Referenzanalytik werden in einem Korrelationsschritt 96 Korrelationsmodelle berechnet. Dazu können die Methoden der Hauptkomponentenregression (PCR) bzw. des linearen bzw. nichtlinearen PLS (Partial Least Square) Verfahren herangezogen.
8. Die mit dem PCR bzw. PLS - Verfahren berechneten Hauptkomponenten können als Eingangsgrößen für ein Neuronales Netz 98 benutzt werden.
9. Aus berechneten Konzentrationen 100a-c des Validiersatzes und den Laborwerten 92a-c werden die statistischen Größen zur Beschreibung des Vorhersagefehlers (RMSEP, RMSECV, BIAS) berechnet. Diese bilden die Grundlagen des optimalen Kalibrationsmodells 88, das für die Applikation im Messsystem 90 eingesetzt wird.
10. Es werden alle Kombinationen (Vorverarbeitung, Normierung, spektrale Bereiche, Korrelationsverfahren) ausprobiert, aus denen das optimale Kalibrationsmodells 88 im Ermittlungsschritt 102 ausgewählt wird.

Das Kalibrationsmodell 88 kann dann aus folgenden Berechnungsvorschriften bestehen:
- Art der Vorverarbeitung
- Angabe der spektralen Bereiche
- Normierungsverfahren und Normierungskoeffizienten
- Art des Korrelationsverfahrens und Hauptkomponenten aus dem PCR bzw. PLS - Verfahren
- Bei Verwendung von ANN - Verfahren die Daten des Neuronalen Netzes.
- Das Ergebnis der Berechnungen ist der Schwefelgehalt C_{S} des Schiffskraftstoffes, z.B. des Diesels 56.

FIG 6 zeigt die Verwendung eines Messsystems 90 an Bord eines Schiffes 52 zur bordeigenen Blendung von Schweröl (HFO) mit Gasöl. Ziel ist die Herstellung eines Brennstoffes 110 mit einem Schwefelgehalt C_{S} von 1,49% zum Einsatz in einem Seegebiet mit erlaubten 1,5% Schwefelgehalt, also z.B. zum Befahren der SECA-Gebiete (Nord- und Ostsee, USA Westküste).

Gezeigt sind ein Schweröl-Servicetank 112, der Treibstoff mit einem Schwefelgehalt C_{S}>>1,5% enthält und ein Gasöl-Servicetank 114 für Treibstoff mit Schwefelgehalt C_{S} im Bereich von 0,2-03,%, mit einem jeweiligen Filter 116a,b.

Beide Tanks sind mit über Mikroprozessor frequenzgesteuerte Pumpen 118a,b mit jeweils nachgeschalteten Durchflussmessern 120a,b für Schweröl Gasöl ausgerüstet.

Beide Tankleitungen münden dann in einen Homogenisator 122 zum Mischen der beiden Treibstoffsorten zum Brennstoff 110. Dem Homogenisator 122 ist dann das Messsystem 90 zur Ermittlung des aktuellen Schwefelgehaltes C_{S} nachgeschaltet. Das Messsystem 90 umfasst - nicht dargestellt - ebenfalls einen nachgeschalteten Durchflussmesser und ein von Behörden anerkanntes schreibendes Anzeigegerät bzw. Protokolliergerät.

Zur Messung des gesamten Volumenstromes des Brennstoffes 110 entlang des Pfeils 111 mit C_{S}=1,49% ist diesem wieder eine Pumpe 124 mit Durchflussmesser, ein Standrohr 126 als Ausgleichsbehälter, eine Pumpe 128 mit Endvorwärmer und Filter nachgeschaltet.

Die Kraftstoffleitung mündet schließlich in einem Viscosimat 130, der die Vorwärmleistung des Endvorwärmers 128 zwecks Erreichung der benötigten Einspritzviskosität bzw. Brennstoffendtemperatur vor den Motoreinspritzpumpen regelt. Der Kraftstoff gelangt dann in den Motor 58. Eine Kombination von Pumpen 118a,b mit Durchflussmessern 120a,b und Homogenisator 122 wurde bereits mit einer Viskositätssonde als Aggregatkombination von der Firma SIT ausgeführt.

## Patentansprüche

1. Kraftstoffsystem für einen Betrieb einer schwimmenden Einrichtung (52), mit einem sich von einem Tankstutzen (54) bis zu einem Motor (58) erstreckenden Kraftstoffweg (50), und mit einer im Kraftstoffweg (50) angeordneten und von zumindest einem Teil des entlang des Kraftstoffweges (50) strömenden Kraftstoffes (56) durchströmbaren Messeinrichtung (90) zur Messung eines Schwefelgehaltes (C_{S}) des die Messeinrichtung (90) durchströmenden Kraftstoffes (56), **gekennzeichnet durch**
- mindestens zwei Kraftstofftanks (112, 114) für Kraftstoffe (56) unterschiedlichen Schwefelgehaltes (C_{S}),
- eine Mischeinrichtung (122) zur Mischung von Kraftstoffen (56) der Kraftstofftanks (112, 114), und eine mit der Messeinrichtung (90) zusammenwirkende Regeleinrichtung zur Regelung des Mischungsverhältnisses der Kraftstoffe.

2. Kraftstoffsystem nach Anspruch 1, bei dem die Messeinrichtung (90) eine Lichtquelle (68) zur Bestrahlung des Kraftstoffes (56) mit Licht (66) und einen Detektor (70) für den Kraftstoff (56) durchstrahlendes oder vom Kraftstoff (56) reflektiertes Licht (66) umfasst.

3. Kraftstoffsystem nach Anspruch 2, bei dem die Lichtquelle (68) eine IR-Lichtquelle ist.

4. Kraftstoffsystem nach Anspruch 2 oder 3 bei dem der Detektor (70) ein IR-Spektrometer ist.

5. Kraftstoffsystem nach einem der Ansprüche 2 bis 4, bei dem die Messeinrichtung (90) eine Auswerteeinheit zur multivariaten Auswertung des den Kraftstoff (56) durchstrahlenden oder vom Kraftstoff (56) reflektierten Lichtes (66) umfasst.

6. Kraftstoffsystem nach einem der vorhergehenden Ansprüche, bei dem die Messeinrichtung (90) in einem zumindest beim Betrieb eines Motors (58) der schwimmenden Einrichtung (52) von Treibstoff (56) durchströmten Abschnittes des Kraftstoffweges (50) angeordnet ist.

7. Kraftstoffsystem nach einem der vorhergehenden Ansprüche, mit einer Protokolliervorrichtung zur Protokollierung von durch die Messeinrichtung (90) ermitteltem Schwefelgehalt (C_{S}).

8. Kraftstoffsystem nach einem der vorhergehenden Ansprüche, bei dem die schwimmende Einrichtung (52) ein Schiff ist.

9. Verfahren zum Betreiben eines Kraftstoffsystems einer schwimmenden Einrichtung (52), wobei dieses einen sich von einem Tankstutzen (54) bis zu einem Motor (58) erstreckenden Kraftstoffweg (50) aufweist, bei dem mit einer im Kraftstoffweg (50) angeordneten und von zumindest einem Teil des entlang des Kraftstoffweges (50) strömenden Kraftstoffes (56) durchströmbaren Messeinrichtung (90) der Schwefelgehalt (C_{S}) im die Messeinrichtung (90) durchströmenden Kraftstoff (56) gemessen wird, **dadurch gekennzeichnet, dass** beim Betrieb der schwimmenden Einrichtung (52) mindestens zwei Kraftstoffe (56) unterschiedlichen Schwefelgehalts (C_{S}) gemischt werden, und bei dem anhand des gemessenen Schwefelgehalts (C_{S}) der Schwefelgehalt (C_{S}) des gemischten Kraftstoffes (56) auf einem vorgebbaren Wert gehalten wird.

10. Verfahren nach Anspruch 9, bei dem der Schwefelgehalt (C_{S}) in der Messeinrichtung (90) mit Hilfe einer IR-Spektroskopie ermittelt wird.

11. Verfahren nach Anspruch 10, bei dem der Schwefelgehalt (C_{S}) aus mit der IR-Spektroskopie ermittelten Spektren (76) durch multivarite Auswertung ermittelt wird.

12. Verfahren nach einem der Ansprüche 9 bis 11, bei dem der Schwefelgehalt (C_{S}) in während des Betriebs einem Motor (58) der schwimmenden Einrichtung (52) zugeführten Kraftstoff (56) ermittelt wird.

13. Verfahren nach einem der Ansprüche 9 bis 12, bei dem der gemessene Schwefelgehalt (C_{S}) protokolliert wird.

## Claims

1. Fuel system for operating a floating unit (52), with a fuel path (50) which extends from a filler neck (54) as far as an engine (58), and with a measuring device (90), which is arranged in the fuel path (50) and through which at least part of the fuel (56) flowing along the fuel path (50) can flow, for measuring a sulfur content (C_{S}) of the fuel (56) flowing through the measuring device (90), **characterized by**
- at least two fuel tanks (112, 114) for fuels (56) of different sulfur content (C_{S}),
- a mixing device (122) for the mixing of fuels (56) of the fuel tanks (112, 114), and a regulating device, cooperating with the measuring device (90), for regulating the mixture ratio of the fuels.

2. Fuel system according to Claim 1, in which the measuring device (90) comprises a light source (68) for irradiating the fuel (56) with light (66) and a detector (70) for light (66) irradiating the fuel (56) or reflected by the fuel (56).

3. Fuel system according to Claim 2, in which the light source (68) is an IR light source.

4. Fuel system according to Claim 2 or 3, in which the detector (70) is an IR spectrometer.

5. Fuel system according to one of Claims 2 to 4, in which the measuring device (90) comprises an evaluation unit for the multivariate evaluation of the light (66) irradiating the fuel (56) or reflected by the fuel (56).

6. Fuel system according to one of the preceding claims, in which the measuring device (90) is arranged in a section of the fuel path (50) through which fuel (56) flows at least during the operation of an engine (58) of the floating unit (52).

7. Fuel system according to one of the preceding claims, with a logging device for logging the sulfur content (C_{S}) determined by the measuring device (90).

8. Fuel system according to one of the preceding claims, in which the floating unit (52) is a ship.

9. Method for operating a fuel system of a floating unit (52), the latter having a fuel path (50) extending from a filler neck (54) as far as an engine (58), in which, by means of a measuring device (90) which is arranged in the fuel path (50) and through which at least part of the fuel (56) flowing along the fuel path (50) can flow, the sulfur content (C_{S}) in the fuel (56) flowing through the measuring device (90) is measured, **characterized in that** at least two fuels (56) of different sulfur content (C_{S}) are mixed during the operation of the floating unit (52), and in which the sulfur content (C_{S}) of the mixed fuel (56) is held at a predeterminable value on the basis of the measured sulfur content (C_{S}).

10. Method according to Claim 9, in which the sulfur content (C_{S}) is determined in the measuring device (90) with the aid of IR spectroscopy.

11. Method according to Claim 10, in which the sulfur content (C_{S}) is determined by a multivariate evaluation from spectra (76) determined by means of the IR spectroscopy.

12. Method according to one of Claims 9 to 11, in which the sulfur content (C_{S}) in fuel (56) supplied during the operation to an engine (58) of the floating unit (52) is determined.

13. Method according to one of Claims 9 to 12, in which the measured sulfur content (C_{S}) is logged.

## Revendications

1. Système à carburant pour le fonctionnement d'un dispositif ( 52 ) flottant, comprenant un trajet ( 52 ) de carburant s'étendant d'un raccord ( 54 ) de cuve jusqu'à un moteur ( 58 ) et comprenant un dispositif ( 90 ) de mesure qui est monté dans le trajet ( 50 ) de carburant et dans lequel peut passer au moins une partie du carburant ( 56 ) passant dans le trajet ( 50 ) de carburant pour la mesure d'une teneur ( Cs ) en soufre du carburant ( 56 ) passant dans le dispositif ( 90 ) de mesure, **caractérisé par**
- au moins deux cuves ( 112, 114 ) de carburant ( 56 ) de teneurs ( Cs ) en soufre différentes,
- un dispositif ( 122 ) de mélange de carburant ( 56 ) des cuves ( 112, 114 ) de carburant et un dispositif de réglage, qui coopère avec le dispositif ( 90 ) de mesure et qui est destiné à régler le rapport de mélange des carburants.

2. Système à carburant suivant la revendication 1, dans lequel le dispositif ( 90 ) de mesure comprend une source ( 68 ) lumineuse pour exposer le carburant ( 56 ) à de la lumière ( 56 ) et un détecteur ( 70 ) de la lumière ( 66 ) traversant le carburant ( 56 ) ou réfléchie par le carburant ( 56 ).

3. Système à carburant suivant la revendication 2, dans lequel la source ( 68 ) lumineuse est une source lumineuse d'infrarouge.

4. Système à carburant suivant la revendication 2 ou 3, dans lequel le détecteur ( 70 ) est un spectromètre d'infrarouge.

5. Système à carburant suivant l'une des revendications 2 à 4, dans lequel le dispositif ( 90 ) de mesure comprend une unité d'exploitation pour l'exploitation multivariée de la lumière ( 66 ) traversant le carburant ( 56 ) ou réfléchie par le carburant ( 56 ).

6. Système à carburant suivant l'une des revendications précédentes, dans lequel le dispositif ( 90 ) de mesure est monté dans un tronçon du trajet ( 50 ) de carburant parcouru par le carburant ( 56 ) au moins pendant le fonctionnement d'un moteur du dispositif ( 52 ) flottant.

7. Système à carburant suivant l'une des revendications précédentes, comprenant un dispositif d'enregistrement pour l'enregistrement de la teneur ( Cs ) en soufre déterminée par le dispositif ( 90 ) de mesure.

8. Système à carburant suivant l'une des revendications précédentes, dans lequel le dispositif ( 52 ) flottant est un bateau.

9. Procédé pour faire fonctionner un système à carburant d'un dispositif ( 52 ) flottant, dans lequel ce système comprend un trajet ( 50 ) de carburant s'étendant d'un raccord ( 54 ) de cuve jusqu'à un moteur ( 58 ), dans lequel on mesure, par un dispositif ( 90 ) de mesure qui est monté dans le trajet ( 50 ) de carburant et dans lequel peut passer au moins une partie du carburant ( 56 ) passant dans le trajet ( 50 ) de carburant, la teneur ( Cs ) en soufre du carburant passant dans le dispositif ( 90 ) de mesure, **caractérisé en ce que**, lorsque le dispositif ( 52 ) flottant fonctionne, on mélange au moins deux carburants ( 56 ) de teneurs ( Cs ) en soufre différentes et dans lequel on maintient, au moyen de la teneur ( Cs ) en soufre mesurée, la teneur ( Cs ) en soufre du carburant ( 56 ) mélangé à une valeur pouvant être prescrite.

10. Procédé suivant la revendication 9, dans lequel on détermine la teneur ( Cs ) en soufre dans le dispositif ( 90 ) de mesure à l'aide d'une spectroscopie infrarouge.

11. Procédé suivant la revendication 10, dans lequel on détermine la teneur ( Cs ) en soufre par exploitation multi variante de spectre ( 76 ) déterminés par spectroscopie infrarouge.

12. Procédé suivant l'une des revendications 9 à 11, dans lequel on détermine la teneur ( Cs ) en soufre dans du carburant ( 56 ) envoyé au dispositif ( 52 ) flottant pendant le fonctionnement d'un moteur ( 58 ).

13. Procédé suivant l'une des revendications 9 à 12, dans lequel on enregistre la teneur ( Cs ) en soufre mesurée.
